# EUROPEAN PATENT SPECIFICATION

(11) **EP 1 951 300 B1**
(45) Date of publication and mention of the grant of the patent: **22.06.2011**
(21) Application number: 06808431.8
(22) Date of filing: 06.11.2006
(51) Int. Cl.: A61K 39/145, A61K 39/39

(54) **CHANGING TH1/TH2 BALANCE IN SPLIT INFLUENZA VACCINES WITH ADJUVANTS**
VERÄNDERUNG DES TH1/TH2-GLEICHGEWICHTS IN SPALT-GRIPPEIMPFSTOFFEN MIT ADJUVANTIEN
MODIFICATION DE L'EQUILIBRE TH1/TH2 DANS DES VACCINS ANTIGRIPPAUX SOUS-UNITAIRES AVEC ADJUVANTS

(30) Priority: 04.11.2005 US 734026 P; 08.06.2006 US 812475 P
(43) Date of publication of application: 06.08.2008
(73) Proprietor: Novartis Vaccines and Diagnostics S.r.l., 53100 Siena (SI) (IT)
(72) Inventor: O'HAGAN, Derek, I-53100 Siena (IT)
(74) Representative: Marshall, Cameron John
(86) International application number: PCT/GB2006/004136
(87) International publication number: WO 2007/052059

(56) References cited:
- WO-A-01/80836
- WO-A-99/27961
- WO-A-2004/075829
- WO-A-2004/084937
- WO-A-2005/117958
- WO-A-2006/060710
- WO-A-2006//100110
- US-A1- 2004 109 874
- S. HALPERIN ET AL.: "Safety and immunogenicity of a new influenza virus grown in mammalian cell culture" VACCINE, vol. 16, no. 13, 1998, pages 1331-1335, XP002428877
- BABIUK J. MED. VIROL. vol. 72, 2004, pages 138 - 142
- KISTNER PLOS ONE vol. 5, no. 2, 2010, page E9349
- COX SCAND. J. IMMUNOL. vol. 69, 2009, pages 576 - 578
- WACK VACCINE 26:552-561 vol. 26, 2008, pages 552 - 561
- TOEWS ANAL. CHIM. ACTA vol. 618, no. 2, 2008, pages 168 - 183
- BABIUK (2004); J. MED. VIROL. 72:138-142,
- KISTNER (2010); PLOS ONE 5(2):E9349,
- COX (2009); SCAND. J. IMMUNOL. 69:576-578,
- WACK (2008); VACCINE 26:552-561,
- TOEWS (2008); ANAL. CHIM. ACTA 618(2):168-183,
- BARACKMAN (1999); INFECT. IMMUN. 67(8):4276-4279,
- WARBURTON (1969); BULL. WORLD HEALTH ORG. 41:639-641,

## Description

### TECHNICAL FIELD

This invention is in the field of vaccines for protecting against influenza virus infection, and in particular split vaccines.

### BACKGROUND ART

Influenza vaccines are described in chapters 17 & 18 of reference 1. They are based on live virus or inactivated virus, and inactivated vaccines can be based on whole virus, 'split' virus or on purified surface antigens (including hemagglutinin and neuraminidase). Haemagglutinin (HA) is the main immunogen in inactivated influenza vaccines, and vaccine doses are standardized by reference to HA levels, with vaccines typically containing about 15µg of HA per strain.

The 'split' vaccines are obtained by treating virions with detergents to produce subvirion preparations, using methods such as the 'Tween-ether' splitting process. Split vaccines generally include multiple antigens from the influenza virion. The BEGRIVAC™, FLUARIX™, FLUZONE™ and FLUSHIELD™ products are split vaccines. WO 01/80836 combines split vaccines with a vector comprising a non liquid hydrophilic core.

During the 2000-01 season in Canada, a newly-identified oculorespiratory syndrome (ORS) was observed in patients who received split vaccines. The ORS has been associated with incomplete splitting of virions during manufacture, giving compositions with a high proportion of microaggregates of unsplit virions [2].

It is an object of the invention to minimize the risk that a split influenza vaccine might elicit ORS.

### DISCLOSURE OF THE INVENTION

There is no causal explanation of the link between split vaccines and ORS, but the clinical and epidemiological features of ORS are suggestive of hypersensitivity, and so it has been proposed that the vaccine may upset the natural Th1/Th2 balance, with the particulate unsplit virions causing a bias towards a Th2 phenotype. In reference 3, for example, the presence of aggregates in split influenza vaccines was found to deviate the immune response to a greater Th2 cytokine pattern. In reference 4, however, no link could be confirmed between ORS and the Th1/Th2 balance.

Despite the lack of any sound evidence linking ORS and a Th2 bias, the invention seeks to minimize the possibility that a split vaccine might cause an excessive Th2 response. In a situation where influenza vaccines have to be produced in a hurry (*e.g.* after a pandemic outbreak) then pressures on manufacturers might inadvertently result in the release of vaccines that suffer from the same problems as the partially-unsplit aggregated Canadian batches from 2000-01. Indeed, reference 2 reports that "it may not be possible to eliminate unsplit virions and aggregates altogether", and that "some low-level risk for triggering ocular and respiratory symptoms may be unavoidable". Accordingly, the invention seeks to avoid components in split vaccines that could cause an excessive Th2 response. Th2 responses are not necessarily avoided altogether, as they can be important for protection, but strong Th2 polarization. If incomplete splitting occurs inadvertently during manufacture, or if a split vaccine undergoes aggregation during storage, any adverse effects *(e.g.* ORS) related to Th2 polarization may be avoided.

To avoid Th2 polarization, two approaches are followed, preferably in combination. Firstly, where a split vaccine includes an adjuvant then the adjuvant is chosen to stimulate at least a partial Th1-type response *e.g.* it is preferred to avoid adjuvanting a split influenza vaccine solely with aluminum salts. Secondly, the presence of allergens is avoided in split vaccines. Because influenza vaccines are unique in being administered every season, patients who receive multiple doses become sensitized to any impurities that are present, and allergens have been reported to cause Th2 responses in sensitized patients [5]. To avoid allergens, the current egg-based methods for growing influenza virus are replaced by methods that use cell culture, thereby avoiding the presence of contaminating egg allergens such as ovalbumin and ovomucoid.

Thus the invention provides an immunogenic composition as defined in claim 1.

The invention also provides a kit as defined in claim 17.

### The split Influenza virus antigen

Compositions of the invention include an antigen obtained by splitting influenza virions that are obtained by viral growth in cell culture. The split virion will typically include multiple antigens from the influenza virion, including hemagglutinin, neuraminidase, matrix and nucleoprotein. The invention does not encompass live virus vaccines (such as the FLUMIST™ product), whole-virion inactivated vaccines (such as the INFLEXAL™ product), purified surface antigen vaccines (which include only the hemagglutinin and neuraminidase surface glycoproteins, such as the FLUVIRIN™, AGREPPAL™ and INFLUVAC™ products) or virosomal vaccines (which take the form of nucleic acid free viral-like liposomal particles [6], as in the INFLEXAL V™ and INVAVAC™ products).

Virions can be harvested from virus-containing fluids by various methods. For example, a purification process may involve zonal centrifugation using a linear sucrose gradient solution that includes detergent to disrupt the virions.

Split virions can be obtained by treating purified virions with detergents (*e.g.* ethyl ether, polysorbate 80, deoxycholate, tri-*N*-butyl phosphate, Triton X-100, Triton N101, cetyltrimethylammonium bromide, Tergitol NP9, *etc.*) to produce subvirion preparations, including the 'Tween-ether' splitting process. Methods of splitting influenza viruses are well known in the art *e.g.* see refs. 7-12, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g.* cationic) surfactants *e.g.* alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N-*butyl phosphate, Cetavlon, myristyltrimethylammonium salts, lipofectin, lipofectamine, and DOT-MA, the octyl- or nonylphenoxy polyoxyethanols (*e.g.* the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (e.g. in a sucrose density gradient solution). Split virions can usefully be resuspended in sodium phosphate-buffered isotonic sodium chloride solution.

The influenza virus may be attenuated. The influenza virus may be temperature-sensitive. The influenza virus may be cold-adapted.

Influenza virus strains used in vaccines change from season to season. In the current inter-pandemic period, trivalent vaccines are typical, including two influenza A strains (H1N1 and H3N2) and one influenza B strain. The invention can be used with inter-pandemic strains of this type, but can also be used with viruses from pandemic strains (*i.e*. strains to which the vaccine recipient and the general human population are immunologically naïve), such as H2, H5, H7 or H9 subtype strains (in particular of influenza A virus), and influenza vaccines for pandemic strains may be monovalent or may, for instance, be based on a normal trivalent vaccine supplemented by a pandemic strain. Depending on the season and on the nature of the antigen included in the vaccine, however, the invention may protect against one or more of influenza A virus HA subtypes H1, H2, H3, H4, H5, H6, H7, H8, H9, H10, H11, H12, H13, H14, H15 or H16. The invention may protect against one or more of influenza A virus NA subtypes N1, N2, N3, N4, N5, N6, N7, N8 or N9.

As well as being suitable for immunizing against inter-pandemic strains, the compositions of the invention are particularly useful for immunizing against pandemic strains. The characteristics of an influenza strain that give it the potential to cause a pandemic outbreak are: (a) it contains a new HA compared to the Has in currently-circulating human strains, *i.e*. one that has not been evident in the human population for over a decade (*e.g.* H2), or has not previously been seen at all in the human population (*e.g.* H5, H6 or H9, that have generally been found only in bird populations), such that the human population will be immunologically naïve to the strain's HA; (b) it is capable of being transmitted horizontally in the human population; and (c) it is pathogenic to humans. A virus with H5 haemagglutinin type is preferred for immunising against pandemic influenza, such as a H5N1 strain. Other possible strains include H5N3, H9N2, H2N2, H7N1 and H7N7, and any other emerging potentially pandemic strains. Within the H5 subtype, a virus may fall into HA clade 1, HA clade 1', HA clade 2 or HA clade 3 [13], with clades 1 and 3 being particularly relevant.

Influenza virus strains used with the invention may be resistant to antiviral therapy (*e.g.* resistant to oseltamivir [14] and/or zanamivir), including resistant pandemic strains [15].

Compositions of the invention may include antigen(s) from one or more (*e.g.* 1, 2, 3, 4 or more) influenza virus strains, including influenza A virus and/or influenza B virus. Where a vaccine includes more than one strain of influenza, the different strains are typically grown separately and are mixed after the viruses have been harvested and split. Thus a process of the disclosure may include the step of mixing split antigens from more than one influenza strain. A trivalent vaccine is preferred, including two influenza A virus strains and one influenza B virus strain.

In some embodiments of the invention, the compositions may include antigen from a single influenza A strain. In some embodiments, the compositions may include antigen from two influenza A strains, provided that these two strains are not H1N1 and H3N2. In some embodiments, the compositions may include antigen from more than two influenza A strains.

The influenza virus may be a reassortant strain, and may have been obtained by reverse genetics techniques. Reverse genetics techniques [*e.g.* 16-20] allow influenza viruses with desired genome segments to be prepared *in vitro* using plasmids. Typically, they involve expressing (a) DNA molecules that encode desired viral RNA molecules *e.g.* from polI promoters, and (b) DNA molecules that encode viral proteins *e.g.* from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA are preferred [21-23], and these methods will also involve the use of plasmids to express all or some (*e.g.* just the PB1, PB2, PA and NP proteins) of the viral proteins, with 12 plasmids being used in some methods.

To reduce the number of plasmids needed, a recent approach [24] combines a plurality of RNA polymerase I transcription cassettes (for viral RNA synthesis) on the same plasmid (*e.g.* sequences encoding 1, 2, 3, 4, 5, 6, 7 or all 8 influenza A vRNA segments), and a plurality of protein-coding regions with RNA polymerase II promoters on another plasmid (*e.g.* sequences encoding 1, 2, 3, 4, 5, 6, 7 or all 8 influenza.A mRNA transcripts). Preferred aspects of the reference 24 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using polI promoters to encode the viral RNA segments, it is possible to use bacteriophage polymerase promoters [25]. For instance, promoters for the SP6, T3 or T7 polymerases can conveniently be used. Because of the species-specificity of polI promoters, bacteriophage polymerase promoters can be more convenient for many cell types (*e.g.* MDCK), although a cell must also be transfected with a plasmid encoding the exogenous polymerase enzyme.

In other techniques it is possible to use dual poll and polII promoters to simultaneously code for the viral RNAs and for expressible mRNAs from a single template [26,27].

Thus an influenza A virus may include one or more RNA segments from a A/PR/8/34 virus (typically 6 segments from A/PR/8/34, with the HA and N segments being from a vaccine strain, *i.e*. a 6:2 reassortant). It may also include one or more RNA segments from a A/WSN/33 virus, or from any other virus strain useful for generating reassortant viruses for vaccine preparation. Typically, the composition of the invention protects against a strain that is capable of human-to-human transmission, and so the strain's genome will usually include at least one RNA segment that originated in a mammalian (*e.g.* in a human) influenza virus. It may include NS segment that originated in an avian influenza virus.

The viruses used as the source of the antigens are grown on cell culture. The viral growth substrate will typically be a cell line of mammalian origin. Suitable mammalian cells of origin include, but are not limited to, hamster, cattle, primate (including humans and monkeys) and dog cells. Various cell types may be used, such as kidney cells, fibroblasts, retinal cells, lung cells, *etc.* Examples of suitable hamster cells are the cell lines having the names BHK21 or HKCC. Suitable monkey cells are *e.g.* African green monkey cells, such as kidney cells as in the Vero cell line. Suitable dog cells are *e.g.* kidney cells, as in the MDCK cell line. Thus suitable cell lines include, but are not limited to: MDCK; CHO; 293T; BHK; Vero; MRC-5; PER.C6; WI-38; *etc.* Preferred mammalian cell lines for growing influenza viruses include: MDCK cells [28-31], derived from Madin Darby canine kidney; Vero cells [32-34], derived from African green monkey (*Cercopithecus aethiops*) kidney; or PER.C6 cells [35], derived from human embryonic retinoblasts. These cell lines are widely available *e.g.* from the American Type Cell Culture (ATCC) collection [36], from the Coriell Cell Repositories [37], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. As a less-preferred alternative to mammalian cell lines, virus can be grown on avian cell lines [*e.g.* refs. 38-40], including cell lines derived from ducks (*e.g.* duck retina) or hens *e.g.* chicken embryo fibroblasts (CEF), *etc.* Examples include avian embryonic stem cells [38, 41], including the EBx cell line derived from chicken embryonic stem cells, EB45, EB14, and EB14-074 [42].

The most preferred cell lines for growing influenza viruses are MDCK cell lines. The original MDCK cell line is available from the ATCC as CCL-34, but derivatives of this cell line may also be used. For instance, reference 28 discloses a MDCK cell line that was_adapted for growth in suspension culture ('MDCK 33016', deposited as DSM ACC 2219). Similarly, reference 43 discloses a MDCK-derived cell line that grows in suspension in serum-free culture ('B-702', deposited as FERM BP-7449). Reference 44 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 45 discloses MDCK cell lines with high susceptibility to infection, including 'MDCK.5F1' cells (ATCC CRL-12042). Any of these MDCK cell lines can be used.

Virus may be grown on cells in suspension [28,46,47] or in adherent culture. As an alternative, microcarrier culture can be used. One suitable MDCK cell line for suspension culture is MDCK 33016 (deposited as DSM ACC 2219).

Cell lines supporting influenza virus replication are preferably grown in serum-free culture media and/or protein free media. A medium is referred to as a serum-free medium in the context of the present invention in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

The cell culture used for growth, and also the viral inoculum used to start the culture, is preferably free from (*i.e*. will have been tested for and given a negative result for contamination by) herpes simplex virus, respiratory syncytial virus, parainfluenza virus 3, SARS coronavirus, adenovirus, rhinovirus, reoviruses, polyomaviruses, bimaviruses, circoviruses, and/or parvoviruses [48]. Absence of herpes simplex viruses is particularly preferred.

Cell lines supporting influenza virus replication are preferably grown below 37°C [49] *e.g.* 30-36°C during viral replication.

Vaccines of the invention preferably contain less than 10ng (preferably less than 1ng, and more preferably less than 100pg) of residual host cell DNA per dose, although trace amounts of host cell DNA may be present. In general, the host cell DNA that it is desirable to exclude from compositions of the invention is DNA that is longer than 100bp.

Measurement of residual host cell DNA is now a routine regulatory requirement for biologicals and is within the normal capabilities of the skilled person. The assay used to measure DNA will typically be a validated assay [50,51]. The performance characteristics of a validated assay can be described in mathematical and quantifiable terms, and its possible sources of error will have been identified. The assay will generally have been tested for characteristics such as accuracy, precision, specificity. Once an assay has been calibrated (*e.g.* against known standard quantities of host cell DNA) and tested then quantitative DNA measurements can be routinely performed. Three principle techniques for DNA quantification can be used: hybridization methods, such as Southern blots or slot blots [52]; immunoassay methods, such as the Threshold™ System [53]; and quantitative PCR [54]. These methods are all familiar to the skilled person, although the precise characteristics of each method may depend on the host cell in question *e.g.* the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [53]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g.* AppTec™ Laboratory Services, BioReliance™, Althea Technologies, *etc*. A comparison of a chemiluminescent hybridisation assay and the total DNA Threshold™ system for measuring host cell DNA contamination of a human viral vaccine can be found in reference 55.

Contaminating DNA can be removed during vaccine preparation using standard purification procedures *e.g.* chromatography, *etc*. Removal of residual host cell DNA can be enhanced by nuclease treatment *e.g.* by using a DNase. A convenient method for reducing host cell DNA contamination is disclosed in references 56 & 57, involving a two-step treatment, first using a DNase (*e.g.* Benzonase), which may be used during viral growth, and then a cationic detergent (*e.g.* CTAB), which may be used during virion disruption. Treatment with an alkylating agent, such as β-propiolactone, can also be used to remove host cell DNA, and advantageously may also be used to inactivate virions [58].

Vaccines containing <10ng (*e.g.* <lng, <100pg) host cell DNA per 15µg of haemagglutinin are preferred, as are vaccines containing <10ng (*e.g.* <1ng, <100pg) host cell DNA per 0.25ml volume. Vaccines containing <10ng (*e.g.* <1ng, <100pg) host cell DNA per 50µg of haemagglutinin are more preferred, as are vaccines containing <10ng (*e.g.* <lng, <100pg) host cell DNA per 0.5ml volume.

The method for propagating virus in cultured cells generally includes the steps of inoculating the cultured cells with the strain to be cultured, cultivating the infected cells for a desired time period for virus propagation, such as for example as determined by virus titer or antigen expression (*e.g.* between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g.* monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture.

Haemagglutinin (HA) is the main immunogen in inactivated influenza vaccines, including in split vaccines, and vaccine doses are standardized by reference to HA levels, typically as measured by a single radial immunodiffusion (SRID) assay. Existing split vaccines typically contain about 15µg of HA per strain, although lower doses are also used *e.g.* for children, or in pandemic situations. Fractional doses such as ½ (*i.e.* 7.5µg HA per strain, and ⅛ have been used [63,64], as have higher doses (*e.g.* 3x or 9x doses [59,60]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 45, about 30, about 15, about 10, about 7.5, about 5, about 3.8, about 1.9, about 1.5, *etc.* per strain. The inclusion of an adjuvant in the vaccine can compensate for the lower inherent immunogenicity of these lower doses.

HA used with the invention may be a natural HA as found in a virus, or may have been modified. For instance, it is known to modify HA to remove determinants (*e.g.* hyper-basic regions around the cleavage site between HA1 and HA2) that cause a virus to be highly pathogenic in avian species.

Compositions of the invention may include detergent *e.g.* a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may included less than 1mg/ml of each of octoxynol-10 and polysorbate 80. Other residual components in trace amounts could be antibiotics (*e.g.* neomycin, kanamycin, polymyxin B).

### The adjuvants

The use of adjuvants with influenza vaccines is known. For instance, the FLUAD™ product, which his based on purified surface antigens, includes a MF59 emulsion adjuvant. Furthermore, references 61 to 64 disclose the use of aluminum salts to adjuvant whole virion influenza vaccines, in order to reduce the amount of antigen required for a single influenza vaccine dose (thereby permitting an increased number of doses to be produced from a fixed amount of antigen). Currently there are no adjuvanted split influenza vaccines on the market.

As aluminum salts (as used in references 61 to 64) promote a Th2-type immune response when used on their own, the invention does not adjuvant split influenza viruses in this way. Instead, alternative adjuvants are used as described below.

The distinction between Th1 and Th2 T helper cells is well known. Th1 and Th2 adjuvants cause an immune response to a co-administered antigen to be biased towards, respectively, a Th1-type or a Th2-type response. Thus Th1 adjuvants result in the production of antigen-specific T cells that release cytokines such as IL-2 and interferon-γ (leading to IgG2a antibodies) and TNF-α, whereas Th2 adjuvants result in the production of antigen-specific T cells that release cytokines such as IL-4 (leading to IgG1) and IL-5. The Th1/Th2 balance of a particular adjuvant can be assessed by known assays (see below), but can vary depending on factors such as the delivery route or the presence of co-administered substances. The adjuvants used with the invention may elicit an exclusively Th1-type response against influenza antigens when delivered to a patient, but will preferably elicit a mixed Th1/Th2-type response. Th0 cells may also be elicited, but a polarized Th2 response will be avoided.

Th1-type responses are naturally linked to bacterial infections, and so adjuvants used with the invention generally include substances that mimic a bacterial substance. Th1 adjuvants may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (*e.g.* imidazoquinolines) and/or TLR9 (*e.g.* CpG oligonucleotides). These agents are useful for activating innate immunity pathways.

### Immunostimulatory oligonucleotides

Immunostimulatory oligonucleotides can include nucleotide modifications/analogs such as phosphorothioate modifications and can be double-stranded or (except for dsRNA) single-stranded. References 111, 112 and 113 disclose possible analog substitutions *e.g,* replacement of guanosine with 2'-deoxy-7-deazaguanosine. The adjuvant effect of CpG oligonucleotides is further discussed in refs. 114-119. The CpG sequence may be directed to TLR9, such as the motif GTCGTT or TTCGTT [120]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 121-123. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 120 & 124-126. A useful CpG adjuvant is CpG7909, also known as ProMune™ (Coley Pharmaceutical Group, Inc.).

As an alternative, or in addition, to using CpG sequences, TpG sequences can be used [127]. These oligonucleotides may be free from unmethylated CpG motifs.

The immunostimulatory oligonucleotide may be pyrimidine-rich. For example, it may comprise more than one consecutive thymidine nucleotide (*e.g.* TTTT, as disclosed in ref. 127), and/or it may have a nucleotide composition with >25% thymidine (*e.g.* >35%, >40%, >50%, >60%, >80%, *etc.).* For example, it may comprise more than one consecutive cytosine nucleotide (*e.g.* CCCC, as disclosed in ref. 127), and/or it may have a nucleotide composition with >25% cytosine (*e.g.* >35%, >40%, >50%, >60%, >80%, *etc.).* These oligonucleotides may be free from unmethylated CpG motifs.

Immunostimulatory oligonucleotides will typically comprise at least 20 nucleotides. They may comprise fewer than 100 nucleotides.

### 3dMPL

3dMPL (also known as 3 de-O-acylated monophosphoryl lipid A or 3-O-desacyl-4'-monophosphoryl, lipid A) is an adjuvant in which position 3 of the reducing end glucosamine in monophosphoryl lipid A has been de-acylated. 3dMPL has been prepared from a heptoseless mutant of *Salmonella minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 103). Preparation of 3dMPL was originally described in reference 129.

3dMPL can take the form of a mixture of related molecules, varying by their acylation (*e.g.* having 3, 4, 5 or 6 acyl chains, which may be of different lengths). The two glucosamine (also known as 2-deoxy-2-amino-glucose) monosaccharides are N-acylated at their 2-position carbons (*i.e,* at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

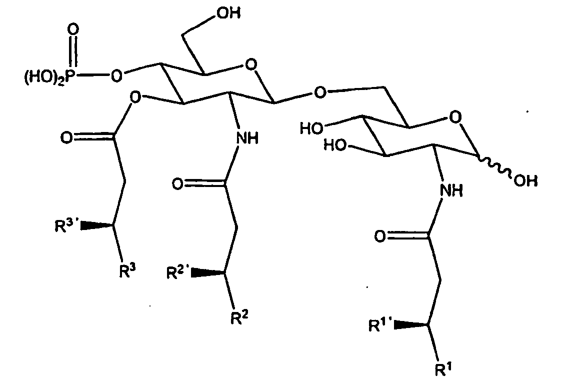

Groups R¹, R² and R³ are each independently -(CH₂)ₙ-CH₃. The value of n is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴, where R⁴ is either -H or -(CH₂)ₘ-CH₃, wherein the value of m is preferably between 8 and 16, and is more preferably 10, 12 or 14. At the 2 position, *m* is preferably 14. At the 2' position, *m* is preferably 10. At the 3' position, m is preferably 12. Groups R¹, R^{2'} and R^{3'} are thus preferably -O-acyl groups from dodecanoic acid, tetradecanoic acid or hexadecanoic acid.

When all of R^{1'}, R^{2'} and R^{3'} are -H then the 3dMPL has only 3 acyl chains (one on each of positions 2, 2' and 3'). When only two of R^{1'}, R^{2'} and R^{3'} are -H then the 3dMPL can have 4 acyl chains. When only one of R^{1'}, R^{2'} and R^{3'} is -H then the 3dMPL can have 5 acyl chains. When none of R^{1'}, R^{2'} and R^{3'} is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL *e.g.* ≥20%, ≥30%, ≥40%, ≥50% or more. 3dMPL with 6 acyl chains has been found to be the most adjuvant-active form.

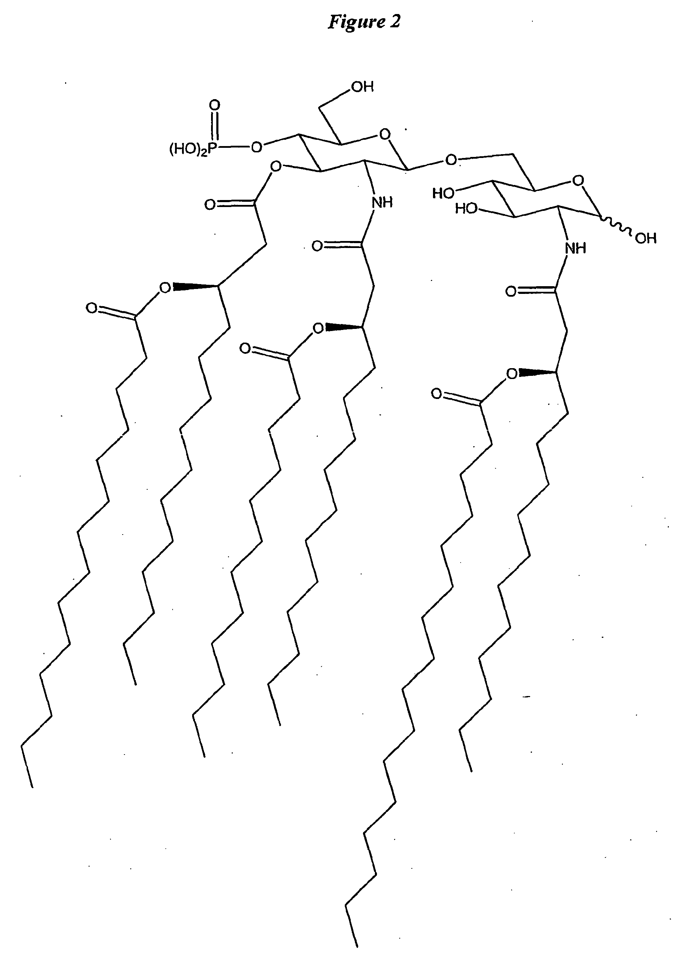

Thus the most preferred form of 3dMPL for inclusion in compositions of the invention is in Figure 2.

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes *e.g.* with a diameter <150nm or >500nm. Either or both of these can be used with the invention, and the better particles can be selected by routine assay. Smaller particles (*e.g.* small enough to give a clear aqueous suspension of 3dMPL) are preferred for use according to the invention because of their superior activity [130]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter of x nm, there will generally be a distribution of particles about this mean, but at least 50% by number (*e.g.* ≥60%, ≥70%, ≥80%, ≥90%, or more) of the particles will have a diameter within the range *x*±25%.

3dMPL can advantageously be used in combination with an oil-in-water emulsion. Substantially all of the 3dMPL may be located in the aqueous phase of the emulsion.

A typical amount of 3dMPL in a vaccine is 10-100µg/dose *e.g.* about 25µg or about 50µg.

### Vitamin E compounds

Reference 135 reports that vitamin E supplementation enhances Th1-type responses. Improvement of humoral and cell-mediated immunity by vitamin E supplementation is also reported in reference 136, but administration as a vaccine adjuvant is reported to have a far greater effect. Moreover, reference 104 reports that vitamin E has a significant impact on the expression of genes involved in the Th1/Th2 balance. For instance, vitamin E stimulation of immune cells can directly lead to increased IL-2 production (*i.e*. a Th1-type response).

Natural vitamin E exists in eight different forms or isomers: four tocopherols and four tocotrienols. All isomers have a chromanol ring, with a hydroxyl group which can donate a hydrogen atom to reduce free radicals and a hydrophobic side chain which allows for penetration into biological membranes. There is an α, β, γ and δ form of both the tocopherols and tocotrienols, determined by the number of methyl groups on the chromanol ring. Each form has its own biological activity, the measure ofpotency or functional use in the body.

Vitamin E compounds for inclusion in compositions of the invention are tocopherols, and any of the α, β, γ, δ, ε or ξ tocopherols can be used. The α-tocopherols are preferred. Advantageous tocopherols have antioxidant properties that may help to stabilize compositions, particularly emulsions [137].

The tocopherols can take several forms *e.g.* different salts and/or isomers. Salts include organic salts, such as succinate, acetate, nicotinate, *etc.* D-α-tocopherol and DL-α-tocopherol can both be used. A preferred α-tocopherol is DL-α-tocopherol, and the preferred salt of this tocopherol is the succinate. Advantageously, α-tocopherol succinate is known to be compatible with influenza vaccines and to be a useful preservative as an alternative to mercurial compounds [11].

As vitamin E compounds are usually oils, they can conveniently be included as a component in an oil-in-water emulsion, as such emulsions are known to be compatible with influenza vaccines (see below), and oil-in-water emulsions containing tocopherols are reported in reference 138 to be Th1-inducing adjuvants.

### Oil-in-water emulsion adjuvants

Oil-in-water emulsions are known to be suitable for adjuvanting influenza virus vaccines *e.g*. the FLUAD™ product contains a MF59 emulsion adjuvant. These emulsions they typically include at least one oil and at least one surfactant, with the oil(s) and surfactant(s) being biodegradable (metabolisable) and biocompatible. The components of the emulsion influence the Th1/Th2 balance, and so not all emulsions are suitable for use with the invention. For instance, the MF59 adjuvant preferentially biases the immune response towards a Th2-type response, and so the invention does not use the MF59 adjuvant on its own (although it can use MF59 in combination with an immunopotentiator, such as an immunostimulatory oligonucleotide). In contrast, the tocopherol-containing emulsions disclosed in reference 138 elicit a Th1-type response, and so can be used with the invention. The Th1/Th2 balance of a particular emulsion can be assessed by conventional assays *e.g.* using the dual-color ELISPOT assays of refs. 139 & 140, the microsphere-based multiplex assay of ref. 141, or the rapid flow cytometric assay of ref.142.

The oil droplets in a suitable emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

Emulsions can include oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g*. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is used herein. Fish oils, including squalene are readily available from commercial sources or may be obtained by methods known in the art. As mentioned above, other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used *e.g.* Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Two specific oil-in-water emulsions used as adjuvants with the invention are:
- An emulsion of squalene, a tocopherol, and Tween 80. The emulsion may include phosphate buffered saline. It may also include Span 85 (*e.g.* at 1%) and/or lecithin. These emulsions may have from 2 to 10% squalene, from 2 to 10% tocopherol and from 0.3 to 3% Tween 80, and the weight ratio of squaleneaocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm.
- A submicron emulsion of squalene, Tween 80, and Span 85 [143-145], also including an immunostimulatory oligonucleotide. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85, as described in more detail in Chapter 10 of ref. 146 and chapter 12 of ref. 147. The emulsion advantageously includes citrate ions *e.g.* 10mM sodium citrate buffer.

These emulsions can be supplemented with 3dMPL and/or with a saponin, as described in reference 138.

Emulsions may be mixed with antigen prior to distribution, or they may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g.* between 5:1 and 1:5) but is generally about 1:1. After the antigen and adjuvant have been mixed, haemagglutinin antigen will generally remain in aqueous solution but may distribute itself around the oil/water interface. In general, little if any haemagglutinin will enter the oil phase of the emulsion.

### Pharmaceutical compositions

Compositions of the invention are pharmaceutically acceptable. They may include components in addition to the split antigen and adjuvant *e.g*. they typically include one or more pharmaceutical carrier(s) and/or excipient(s). A thorough discussion of such components is available in ref. 148.

Compositions will generally be in aqueous form. The split antigen and adjuvant will typically be in admixture.

The composition may include preservatives such as thiomersal or 2-phenoxyethanol. It is preferred, however, that the vaccine should be substantially free from (*i.e.* less than 5µg/ml) mercurial material *e.g.* thiomersal-free [11,149]. Vaccines containing no mercury are more preferred, and this can conveniently be achieved when using a tocopherol-containing adjuvant by following ref. 11. Preservative-free vaccines are particularly preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [150], but keeping osmolality in this range is nevertheless preferred.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range. An emulsion formed in phosphate-buffered saline can conveniently be used.

The pH of a composition will generally be between 5.0 and 8.1, and more typically between 6.0 and 8.0 *e.g*. 6.5 and 7.5, or between 7.0 and 7.8. A process of the invention may therefore include a step of adjusting the pH of the bulk vaccine prior to packaging.

The composition is preferably sterile. The composition is preferably non-pyrogenic *e.g*. containing <1 EU (endotoxin unit, a standard measure) per dose, and preferably <0.1 EU per dose. The composition is preferably gluten free.

The composition may include material for a single immunisation, or may include material for multiple immunisations (*i.e*. a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose (*i.e*. about 0.25ml) may be administered *e.g*. to children.

Compositions and kits are preferably stored at between 2°C and 8°C. They should not be frozen. They should ideally be kept out of direct light.

### Kits of the invention

Compositions of the invention may be prepared extemporaneously, at the time of delivery. Thus the invention provides kits including the various components ready for mixing. The kit allows the adjuvant and the antigen to be kept separately until the time of use, which can be useful when using an oil-in-water emulsion adjuvant.

The components are physically separate from each other within a kit, and this separation can be achieved in various ways. For instance, the two components may be in two separate containers, such as vials. The contents of the two vials can then be mixed *e.g*. by removing the contents of one vial and adding them to the other vial, or by separately removing the contents of both vials and mixing them in a third container.

In a preferred arrangement, one of the kit components is in a syringe and the other is in a container such as a vial. The syringe can be used (*e.g*. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe *e.g.* a dual-chamber syringe, such as those disclosed in references 151-158 *etc.* When the syringe is actuated (*e.g*. during administration to a patient) then the contents of the two chambers are mixed. This arrangement avoids the need for a separate mixing step at the time of use.

The kit components will generally be in aqueous form. In some arrangements, a component (typically the antigen component rather than the adjuvant component) is in dry form *(e.g.* in a lyophilised form), with the other component being in aqueous form. The two components can be mixed in order to reactivate the dry component and give an aqueous composition for administration to a patient. A lyophilised component will typically be located within a vial rather than a syringe. Dried components may include stabilizers such as lactose, sucrose or mannitol, as well as mixtures thereof *e.g.* lactose/sucrose mixtures, sucrose/mannitol mixtures, *etc.* One possible arrangement uses an aqueous adjuvant component in a pre-filled syringe and a lyophilised antigen component in a vial.

### Packaging of compositions or kit components

Suitable containers for compositions of the invention (or kit components) include vials, syringes (*e.g*. disposable syringes), nasal sprays, *etc.* These containers should be sterile.

Where a composition/component is located in a vial, the vial may be made of a glass or plastic material. It can be sterilized before the composition/component is added to it. To avoid problems with latex-sensitive patients, vials may be sealed with a latex-free stopper, and the absence of latex in all packaging material is preferred. The vial may include a single dose of vaccine, or it may include more than one dose (a 'multidose' vial) *e.g*. 10 doses. Preferred vials are made of colorless glass.

A vial can have a cap (*e.g*. a Luer lock) adapted such that a pre-filled syringe can be inserted into the cap, the contents of the syringe can be expelled into the vial (*e.g*. to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"^{™}.

Containers may be marked to show a half-dose volume *e.g.* to facilitate delivery to children. For instance, a syringe containing a 0.5ml dose may have a mark showing a 0.25ml volume.

Where a glass container (*e.g*. a syringe or a vial) is used, then it is preferred to use a container made from a borosilicate glass rather than from a soda lime glass.

A kit or composition may be packaged (*e.g*. in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.*

### Methods of treatment, and administration of the vaccine

Compositions of the invention are suitable for administration to human patients in a method of raising an immune response in a patient, comprising the step of administering a composition of the invention to the patient.

The immune response raised by these methods will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [159]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (*e.g*. into the ann or leg), but other available routes include subcutaneous injection, intranasal [160-162], oral [163], intradermal [164,165], transcutaneous, transdermal [166], *etc*.

Compositions of the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g*. ≥50 years old, ≥60 years old, preferably ≥65 years), the young (*e.g.* ≤5 years old), hospitalised patients, healthcare workers, armed service and military personnel, pregnant women, the chronically ill, immunodeficient patients, patients who have taken an antiviral compound (*e.g.* an oseltamivir or zanamivir compound; see below) in the 7 days prior to receiving the vaccine, people with egg allergies and people travelling abroad. The vaccines are not suitable solely for these groups, however, and may be used more generally in a population. For pandemic strains, administration to all age groups is preferred.

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Administration of more than one dose (typically two doses) is particularly useful in immunologically naïve patients *e.g.* for people who have never received an influenza vaccine before, or for vaccinating against a new HA subtype (as in a pandemic outbreak). Multiple doses will typically be administered at least 1 week apart (*e.g*. about 2 weeks, about 3 weeks, about 4 weeks, about 6 weeks, about 8 weeks, about 10 weeks, about 12 weeks, about 16 weeks, *etc.*)*.*

Vaccines of the invention may be administered to patients at substantially the same time as (*e.g*. during the same medical consultation or visit to a healthcare professional or vaccination centre) other vaccines *e.g*. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H. influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as *(e.g.* during the same medical consultation or visit to a healthcare professional) an antiviral compound, and in particular an antiviral compound active against influenza virus (*e.g*. oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g*. the ethyl esters) and salts thereof (*e.g*. the phosphate salts). A preferred antiviral is (3R,4R,SS)-4-acetylamino-5-amino-3(1-ethylpropoxy)-1-cyclohexene-1-carboxylic acid, ethyl ester, phosphate (1:1), also known as oseltamivir phosphate (TAMIFLU™).

### General

The term "comprising" encompasses "including" as well as "consisting" *e.g*. a composition "comprising" X may consist exclusively of X or may include something additional *e.g*. X + Y.

The word "substantially" does not exclude "completely" *e.g*. a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials. Where a compound is administered to the body as part of a composition then that compound may alternatively be replaced by a suitable prodrug.

Where a cell substrate is used for reassortment or reverse genetics procedures, it is preferably one that has been approved for use in human vaccine production *e.g.* as in Ph Eur general chapter 5.2.3.

### BRIEF DESCRIPTION OF DRAWING

Figure 1 shows the number of cytokine positive cells, as a % of total CD4+ cells. Responses from two individual mice are shown. Mice were immunized with split vaccines "A" or "B", either unadjuvanted or adjuvanted with (i) alum or (ii) MF59 with a CpG oligonucleotide.
Figure 2 shows the formula of the most preferred form of 3dMPL for use with the invention.

### MODES FOR CARRYING OUT THE INVENTION

### Oil-in-water emulsion adjuvant favouring Th1 response

Two commercially available unadjuvanted split virion trivalent influenza vaccines ("SPLIT (A)" and "SPLIT (B)") were obtained and used to immunize mice at a dose of 0.2µg HA. Vaccines were used either unadjuvanted, or adjuvanted with (i) aluminium hydroxide or (ii) a mixture of a MF59 emulsion and 10µg of an immunostimulatory CpG oligonucleotide. Groups of 8 female Balb/C mice, 8 weeks old, were immunized intramuscularly with the vaccines, with 50µl doses on days 0 and 28. Sera were obtained on days 14 and 42, and were analysed for anti-HA titer (IgG), HI titer and T cells.

Serum IgG antibody titers (ELISA) at day 42 were as follows, looking at each virus separately:

| | **No adjuvant** | **Alum** | **MF59+CpG** |
|---|---|---|---|
| **A*nti-H1N1*** | | | |
| SPLIT (A) | 749 | 1329 | 8808 |
| SPLIT (B) | 1175 | 1991 | 6754 |

| ***Anti-H3N2*** | | | |
|---|---|---|---|
| SPLIT (A) | 412 | 977 | 6032 |
| SPLIT (B) | 1111 | 1465 | 5308 |

| ***Anti-B*** | | | |
|---|---|---|---|
| SPLIT (A) | 707 | 2534 | 11211 |
| SPLIT (B) | 1585 | 2520 | 10837 |

HI serum antibody titers at day 42 were as follows:

| | **No adjuvant** | **Alum** | **MF59+CpG** |
|---|---|---|---|
| ***Anti-H1N1*** | | | |
| SPLIT (A) | 140 | 280 | 1387 |
| SPLIT (B) | 285 | 330 | 1371 |

| ***Anti-H3N2*** | | | |
|---|---|---|---|
| SPLIT (A) | 290 | 780 | 800 |
| **SPLIT (B)** | 380 | 440 | 1371 |

| ***Anti-B*** | | | |
|---|---|---|---|
| SPLIT (A) | 280 | 780 | 800 |
| SPLIT (B) | 550 | 440 | 1371 |

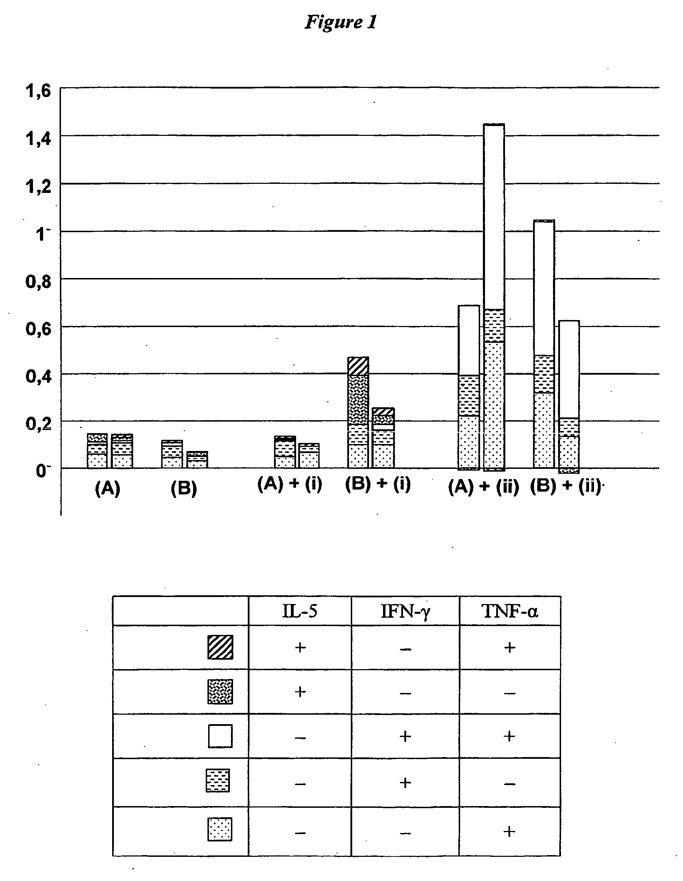

As shown in Figure 1, unadjuvanted vaccines elicited very low levels of antigen-specific T cells. The alum adjuvant increased levels, but in a Th2-biased manner: in one mouse alum caused a large increase in IL-5⁺IFN-γ⁻TNF-α⁻ T cells (*i.e*. Th2-type), but IL-5-IFN-γ⁺TNF-α⁺ cells (*i.e*. Th1-type were not seen. In contrast, a combination of MF59 and a CpG oligonucleotide caused both a large increase in the number of antigen-specific T cells and also a shift towards a Th1-type response.

Thus it is possible to use an oil-in-water emulsion adjuvant to raise the number of antigen-specific T cells elicited by a split influenza vaccine, and also to shift the immune response towards a Th1-type response. In contrast, an alum adjuvant elicits low levels of T cells with a Th2-type response.

### Human trials [reference 167]

As described in reference 167, split influenza vaccines were adjuvanted with an oil-in-water emulsion having an organic phase made of two oils (α-tocopherol and squalene), and an aqueous phase of phosphate buffered saline (PBS) containing Tween 80 as emulsifying agent. It has a final concentration of 2.5% squalene (v/v), 2.5% α-tocopherol (v/v), and 0.9% polyoxyethylene sorbitan monooleate (v/v) (Tween 80). It was initially prepared as a two-fold concentrate for mixing with influenza antigens. This emulsion is reported to be a Th1-inducing adjuvant.

The emulsion was prepared by dissolving Tween 80 is (PBS) to give a 2% solution. To provide 100ml of two-fold concentrate, 5 g of D,L-α-tocopherol and 5ml of squalene were vortexed to mix them thoroughly. 90ml of the PBS/Tween solution was added to the oil mixture and mixed thoroughly. The resulting emulsion was then passed through a syringe and finally microfluidised . The resulting oil droplets have a size of approximately 120-180 nm (Z average).

In control experiments, the emulsion was not included.

Trivalent vaccines were administered to elderly human patients. Humoral and cell-mediated immune responses were measured in the patients as described in reference 167. Seroprotection and seroconversion were determined.

Seroprotection and seroconversion rates were as follows (left = control; right =adjuvanted):

| | **A/New Caledonia** | | **A/Panama** | | **B/Shangdong** | |
|---|---|---|---|---|---|---|
| **Seroprotection** | 98.0 | 98.0 | 91.8 | 100.0 | 95.9 | 100.0 |
| **Seroconversion** | 69.4 | 69.4 | 65.3 | 55.1 | 69.4 | 73.5 |

Anti-HA antibody responses were as follows:

| **Day** | **A/New Caledonia** | **A/Panama** | **B/Shangdong** |
|---|---|---|---|
| ***Control: unadjuvanted*** | | | |
| **0** | 26.3 | 40.9 | 26.0 |
| **21** | 358.5 | 296.0 | 270.0 |

| ***Emulsion-adjuvanted*** | | | |
|---|---|---|---|
| **0** | 25.6 | 52.3 | 27.5 |
| **21** | 317.7 | 366.1 | 317.7 |

To compare Th1 and Th2 responses between the control unadjuvanted vaccine and the emulsion-adjuvanted vaccine, cytokine responses were assayed. CD4+ T cells taken from immunized patients were re-stimulated with a split antigen and were assessed for the number secreting (i) at least interferon-γ (indicative of a Th1 response) and one other of IL-2, TNFα or CD40L; or (ii) at least IL-2 (again, indicative of a Th1 response) and one other of IFN-γ, TNFα or CD40L. Results, expressed as a difference before and after immunization, were as follows:

| | **Unadjuvanted** | | **Adjuvanted** | |
|---|---|---|---|---|
| | **IFN-γ** | **IL-2** | **IFN-γ** | **IL-2** |
| **CD4** | 1149 | 1738 | 2712 | 3465 |

The adjuvanted vaccine shows more of a Th1-type response than the unadjuvanted control. Thus the tocopherol-containing adjuvant is able to modulate the Th1/Th2 balance of a split influenza vaccine.

It will be understood that the invention has been described by way of example only and modifications may be made whilst remaining within the scope of the invention.

### REFERENCES

[1] Vaccines. (eds. Plotkin & Orenstein). 4th edition, 2004, ISBN: 0-7216-9688-0.
[2] Scheifele et al. (2003) Clin Infect Dis 36:850-7.
[3] Babiuk et al. (2004) J Med Virol 72:138-42.
[4] Skowronski et al. (2003) J Infect Dis 187:495-9.
[5] Jenmalm et al. (2001) Clin Exp Allergy 31:1528-35.
[6] Huckriede et al. (2003) Methods Enzymol 373:74-91.
[7] WO02/28422.
[8] WO02/067983.
[9] WO02/074336.
[10] WO01/21151.
[11] WO02/097072.
[12] W02005/113756.
[13] World Health Organisation (2005) Emerging Infectious Diseases 11(10):1515-21.
[14] Herlocher et al. (2004) J Infect Dis 190(9):1627-30.
[15] Le et al. (2005) Nature 437(7062):1108.
[16] Hoffmann et al. (2002) Vaccine 20:3165-3170.
[17] Subbarao et al. (2003) Virology 305:192 200.
[18] Liu et al. (2003) Virology 314:580-590.
[19] Ozaki et al. (2004) J. Virol. 78:1851-1857.
[20] Webby et al. (2004) Lancet 363:1099-1103.
[21] WO00/60050.
[22] WO01/04333.
[23] US patent 6649372.
[24] Neumann et al. (2005) Proc Natl Acad Sci USA 102:16825-9.
[25] WO2006/067211.
[26] WO01/83794.
[27] Hoffmann et al. (2000) Virology 267(2):310-7.
[28] WO97/37000.
[29] Brands et al. (1999) Dev Biol Stand 98:93-100.
[30] Halperin et al. (2002) Vaccine 20:1240-7.
[31] Tree et al. (2001) Vaccine 19:3444-50.
[32] Kistner et al. (1998) Vaccine 16:960-8.
[33] Kistner et al. (1999) Dev Biol Stand 98:101-110.
[34] Bruhl et al. (2000) Vaccine 19:1149-58.
[35] Pau et al. (2001) Vaccine 19:2716-21.
[36] *http:*//*www.atcc.orgl*
[37] *http.*//*locus.umdnj.edu*/
[38] WO03/076601.
[39] WO2005/042728.
[40] WO03/043415.
[41] WO01/85938.
[42] WO2006/108846.
[43] EP-A-1260581 (WO01/64846).
[44] W02006/071563.
[45] WO2005/113758.
[46] WO03/023021
[47] WO03/023025
[48] WO2006/027698.
[49] WO97/37001.
[50] Lundblad (2001) Biotechnology and Applied Biochemistry 34:195-197.
*[51]* Guidance for Industry: Bioanalytical Method Validation. U.S. Department of Health and Human Services Food and Drug Administration Center for Drug Evaluation and Research (CDER) Center for Veterinary Medicine (CVM). May 2001.
[52] Ji et al. (2002) Biotechniques. 32:1162-7.
[53] Briggs (1991) J Parenter Sci Technol. 45:7-12.
[54] Lahijani et al. (1998) Hum Gene Ther. 9:1173-80.
[55] Lokteff et al. (2001) Biologicals. 29:123-32.
[56] EP-B-0870508.
[57] US patent 5948410.
[58] International patent application entitled "CELL-DERIVED VIRAL VACCINES WITH LOW LEVELS OF RESIDUAL CELL DNA", filed 1st November 2006 claiming priority US-60/732786.
[59] Treanor et al. (1996) J Infect Dis 173:1467-70.
[60] Keitel et al. (1996) Clin Diagn Lab Immunol 3:507-10.
[61] US patent 6,372,223.
[62] WO00/15251.
[63] WO01/22992.
[64] Hehme et al. (2004) Virus Res. 103(1-2):163-71.
[65] Cooper et al. (2004) Vaccine 23(2):236-46. :
[66] Dentl et al. (1999) Clin Chem Lab Med 37(3):199-204.
[67] Heeg & Zimmerman (2000) Int Arch Allergy Immunol. 121(2):87-97.
[68] Myers et al. (1990) pages 145-156 of Cellular and molecular aspects of endotoxin reactions*.*
[69] Ulrich (2000) Chapter 16 (pages 273-282) of reference 109.
[70] Johnson et al. (1999) J Med Chem 42:4640-9.
[71] Baldrick et al. (2002) Regulatory Toxicol Pharmacol 35:398-413.
[72] Wheeler et al. (2001) International Archives of Allergy and Immunology 126:135-139
[73] US 5,057,540.
[74] WO96/33739.
[75] EP-A-0109942.
[76] WO96111711.
[77] WO00/07621.
[78] Barr et al. (1998) Advanced Drug Delivery Reviews 32:247-271.
[79] Sjolanderet et al. (1998) Advanced Drug Delivery Reviews 32:321-338.
[80] Pizza et al. (2000) Int J Med Microbiol 290:455-461*.*
[81] WO95/17211.
[82] WO8/42375.
[83] Feng et al. (2005) Acta Biochim Biophys Sin (Shanglrai). 37(2):126-32. ,
[84] Barackman et al. (1999) Infect Immun 67(8):4276-9.
[85] Singh et al. (1998) Vaccine 16(19):1822-7.
[86] Rosenkrands et al. (2005) Infect Immun 73(9):5817-26.
[87] US patent 6355271.
[88] He et al. (2000) Clin Diagn Lab Immunol 7(6): 899-903.
[89] WO2004/064715.
[90] Cooper (1995) Pharm Biotechnol 6:559-80.
[91] Silva et al. (2004) Immunol Cell Biol 82(6):611-6.
[92] US 4,680,338.
[93] US 4,988,815.
[94] WO92/15582..
[95] Stanley (2002) Clin Exp Dermatol 27:571-577.
[96] Wu et al. (2004) Antiviral Res. 64(2):79-83.
[97] Vasilakos et al. (2000) Cell Immunol. 204(1):64-74.
[98] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[99] Jones (2003) Curr Opin Investig Drugs 4:214-218.
[100] US patent 5,011,828.
[101] Johnson et al. (1999) Bioorg Med Chem Lett 9:2273-2278.
[102] Evans et al. (2003) Expert Rev Vaccines 2:219-229.
[103] Thompson et al. (2005) Journal of Leukocyte Biology 78:1273-1280
[104] Han et al. (2004) Ann N Y Acad Sci 1031:96-101.
[105] Franchini et al. (1995) Poult Sci 74(4):666-71.
[106] Wong et al. (2003) J Clin Pharmacol 43(7):735-42.
[107] US2005/0215517.
[108] Vaccine Design: The Subunit and Adjuvant Approach (eds. Powell & Newman) Plenum Press 1995 (ISBN 0-306-44867-X).
[109] Vaccine Adjuvants: Preparation Methods and Research Protocols (Volume 42 of Methods in Molecular Medicine series). ISBN: 1-59259-083-7. Ed. O'Hagan.
[110] Moore et al. (1999) Vaccine 17(20-21):2517-27.
[111] Kandimalla et al. (2003) Nucleic Acids Research 31:2393-2400.
[112] WO02/26757.
[113] WO99/62923.
[114] Krieg (2003) Nature Medicine 9:831-835.
[115] McCluskie et al. (2002) FEMS Immunology and Medical Microbiology 32:179-185.
[116] WO98/40100.
[117] US 6,207,646.
[118] US 6,239,116.
[119] US 6,429,199.
[120] Kandimalla et al. (2003) Biochemical Society Transactions 31 (part 3):654-658.
[121] Blackwell et al. (2003) J Immunol 170:4061-4068.
[122] Krieg (2002) Trends Immunol 23:64-65.
[123] WO01/95935.
[124] Kandimalla et al. (2003) BBRC 306:948-953.
[125] Bhagat et al. (2003) BBRC 300:853-861.
[126] WO03/035836.
[127] WO01/22972.
[128] Jiao et al. (2004) J Gen Virol 85(Pt 6):1545-53.
[129] UK patent application GB-A-2220211.
[130] WO94/21292.
[131] WO94/00153.
[132] WO95/17210.
[133] WO96/26741.
[134] WO93/19780.
[135] Long & Santos (1999) Pediatr Infect Dis J 18:283-90.
[136] Tengerdy (1989) Ann N Y Acad Sci 570:335-44.
[137] US-6630161.
[138] WO02/38176.
[139] Okamoto & Nishida (2005) Methods Mol Biol 302:263-72.
[140] Okamoto et al. (2004) Int Immunopharmacol 4(1):149-56.
[141] Prabhakar et al. (2004) J Immunol Methods 291(1-2):27-38.
[142] Farrell et al. (2001) Br J Dermatol 144(1):24-33.
[143] WO90/14837.
[144] Podda & Del Giudice (2003) Expert Rev Vaccines 2:197-203.
[145] Podda (2001) Vaccines 19: 2673-2680.
[146] Vaccine Design: The Subunit and Adjuvant Approach (eds. Powell & Newman) Plenum Press 1995 (ISBN 0-306-44867-X).
[147] Vaccine Adjuvants: Preparation Methods and Research Protocols (Volume 42 of Methods in Molecular Medicine series). ISBN: 1-59259-083-7. Ed. O'Hagan.
[148] Gennaro (2000) Remington: The Science and Practice of Pharmacy. 20th edition, ISBN: 0683306472.
[149] Banzhoff (2000) Immmology Letters 71:91-96.
[150] Nony et al. (2001) Vaccine 27:3645-51.
[151] WO2005/089837.
[152] US patent 6,692,468.
[153] WO00/07647.
[154] WO99/17820.
[155] US patent 5,971,953.
[156] US patent 4,060,082.
[157] EP-A-0520618.
[158] WO98/01174.
[159] Potter & Oxford (1979) Br Med Bull 35: 69-75.
[160] Greenbaum et al. (2004) Vaccine 22:2566-77.
[161] Zurbriggen et al. (2003) Expert Rev Vaccines 2:295-304.
[162] Piascik (2003) J Am Pharm Assoc (Wash DC). 43:728-30.
[163] Mann et al. (2004) Vaccine 22:2425-9.
[164] Halperin et al. (1979) Am J Public Health 69:1247-50.
[165] Herbert et al. (1979) J Infect Dis 140:234-8.
[166] Chen et al. (2003) Vaccine 21:2830-6.
[167] WO2006/100109.

## Claims

1. An immunogenic composition comprising a split influenza virus antigen and a Th1 adjuvant, wherein the antigen is prepared from a virus grown in cell culture and does not include any egg proteins and the Th1 adjuvant is in the form of (i) an oil-n-water emulsion which includes squalene, a tocopherol,and polysorbate 80, or (ii) a submicron emulsion of squalene, polysorbate 80, sorbitan trioleate, and an immunostimulatory oligonucleotide.

2. The composition of claim 1, wherein the influenza virus antigen is from a H1, H2, H3, H5, H7 or H9 influenza A virus subtype.

3. The composition of claim 1 or claim 2, wherein the composition is free from ovalbumin, ovomucoid and chicken DNA.

4. The composition of any preceding claim, wherein the composition contains between 0.1 and 20µg of haemagglutinin per viral strain.

5. The composition of claim 4, wherein the composition contains about 15µg of HA per strain or contains about 3.8µg of HA per strain.

6. The composition of any preceding claim, wherein the tocopherol is DL-α-tocopherol.

7. The composition of any preceding claim, wherein the emulsion has droplets with a sub-micron diameter.

8. The composition of any preceding claim, wherein the influenza virus antigen is prepared from an influenza virus having one or more RNA segments from an A/PR/8/34 influenza virus.

9. The composition of any preceding claim, wherein the adjuvant comprises a 3-O-deacylated monophosphoryl lipid A (3dMPL).

10. The composition of claim 16, wherein at least 10% by weight of the 3dMPL is the hexaacyl chain form.

11. The composition of any preceding claim, being substantially free from mercurial material.

12. The composition of any preceding claim, including one or more buffer(s).

13. The composition of claim 12, wherein the buffer(s) include: a phosphate buffer; a Tris buffer; a borate buffer; a succinate buffer; a histidine buffer; or a citrate buffer.

14. The composition of any preceding claim, having a pH between 5.0 and 8.1.

15. The composition of any preceding claim, wherein the composition includes two influenza A strains and one influenza B strain.

16. The composition of any one of claims 1 to 14, wherein the composition is a monovalent vaccine against a pandemic influenza virus strain.

17. A kit for preparing the composition of any preceding claim, comprising (a) a first kit component comprising a split influenza virus antigen prepared from a virus grown in cell culture and not including any egg proteins, and (b) a second kit component comprising a Th1 adjuvant in the form of (i) an -oil-in-water emulsion which includes squalene, a tocopherol, and polysorbate 80, or (ii) a submicron emulsion of squalene, polysorbate 80, sorbitan trioleate, and an immunostimulatory oligonucleotide; wherein the first and the second component are in separate containers.

## Patentansprüche

1. Immunogene Zusammensetzung, umfassend ein Spalt-Grippevirus-Antigen und ein Th1-Adjuvans, wobei das Antigen aus einem in Zellkultur gewachsenen Virus präpariert wird und keine Eiproteine enthält und das Th1-Adjuvans in Form (i) einer Öl-in-Wasser-Emulsion, die Squalen, ein Tocopherol und Polysorbat 80 enthält, oder (ii) einer Submikron-Emulsion von Squalen, Polysorbat 80, Sorbitantrioleat und einem immunstimulierenden Oligonukleotid vorliegt.

2. Zusammensetzung nach Anspruch 1, wobei das Grippevirus-Antigen aus einem H1-, H2-, H3-, H5-, H7- oder H9-Influenza-A-Virus-Subtyp stammt.

3. Zusammensetzung nach Anspruch 1 oder Anspruch 2, wobei die Zusammensetzung frei von Ovalbumin, Ovomucoid und Hühner-DNA ist.

4. Zusammensetzung nach einem vorhergehenden Anspruch, wobei die Zusammensetzung zwischen 0,1 und 20 µg Hämagglutinin pro Virusstamm enthält.

5. Zusammensetzung nach Anspruch 4, wobei die Zusammenserzung etwa 15 µg HA pro Stamm oder etwa 3,8 µg HA pro Stamm enthält.

6. Zusammensetzung nach einem vorhergehenden Anspruch, wobei es sich bei dem Tocopherol um DL-α-Tocopherol handelt.

7. Zusammensetzung nach einem vorhergehenden Anspruch, wobei die Emulsion Tröpfchen mit einem Submikron-Durchmesser aufweist.

8. Zusammensetzung nach einem vorhergehenden Anspruch, wobei das Grippevirus-Antigen aus einem Grippevirus mit einem oder mehreren RNA-Segmenten aus einem A/PR/8/34-Influenza-Virus präpariert wird.

9. Zusammensetzung nach einem vorhergehenden Anspruch, wobei das Adjuvans ein 3-O-deacyliertes Monophosphoryllipid A (3dMPL) umfasst.

10. Zusammensetzung nach Anspruch 9, wobei es sich bei wenigstens 10 Gew.-% des 3dMPL um die Hexaacylkettenform handelt.

11. Zusammensetzung nach einem vorhergehenden Anspruch, weitgehend frei von Quecksilbermaterial.

12. Zusammensetzung nach einem vorhergehenden Anspruch, enthaltend einen oder mehrere Puffer.

13. Zusammensetzung nach Anspruch 12, wobei zu dem Puffer bzw. den Puffern ein Phosphatpuffer, ein Tris-Puffer, ein Boratpuffer, ein Succinatpuffer, ein Histidinpuffer oder ein Citratpuffer zählt.

14. Zusammensetzung nach einem vorhergehenden Anspruch mit einem pH-Wert zwischen 5,0 und 8,1.

15. Zusammensetzung nach einem vorhergehenden Anspruch, wobei die Zusammensetzung zwei Influenza-A-Stämme und einen Influenza-B-Stamm enthält.

16. Zusammensetzung nach einem der Ansprüche 1 bis 14, bei der es sich um einen einwertigen Impfstoff gegen einen pandemischen Grippevirusstamm handelt.

17. Kit zur Herstellung einer Zusammensetzung nach einem vorhergehenden Anspruch, umfassend (a) eine erste Kitkomponente, die ein aus einem in Zellkultur gewachsenen Virus präpariertes Spalt-Grippevirus-Antigen umfasst wird und keine Eiproteine enthält, und (b) eine zweite Kitkomponente, die ein Th1-Adjuvans in Form (i) einer Öl-in-Wasser-Emulsion, die Squalen, ein Tocopherol und Polysorbat 80 enthält, oder (ii) einer Submikron-Emulsion von Squalen, Polysorbat 80, Sorbitantrioleat und einem immunstimulierenden Oligonukleotid enthält; wobei die erste und die zweite Komponente in getrennten Behältern vorliegen.

## Revendications

1. Composition immunogène comprenant un antigène sous-unitaire du virus de la grippe et un adjuvant Th1, **caractérisée en ce que** l'antigène est préparé à partir d'un virus cultivé dans une culture cellulaire et ne comporte aucune protéine d'oeuf et l'adjuvant Th1 est sous forme (i) d'une émulsion huile dans l'eau qui comporte du squaléne, un tocophérol et un polysorbate 80, ou (ii) une émulsion sub-micronique de squalène, de polysorbate 80, de trioléate de sorbitane, et d'un oligonucléotide immunostimulateur.

2. Composition selon la revendication 1, **caractérisée en ce que** l'antigène du virus de la grippe vient d'un virus de la grippe A de sous-type H1, H2, H3, H5, H7 ou H9.

3. Composition selon la revendication 1 ou la revendication 2, **caractérisée en ce que** la composition est dépourvue d'ovalbumine, d'ovomucoïde et d'ADN de poulet.

4. Composition selon l'une quelconque des revendications précédentes **caractérisée en ce que** la composition contient entre 0,1 et 20 µg d'hémaglutinine par souche virale.

5. Composition selon la revendication 4, **caractérisée en ce que** la composition contient environ 15 µg de HA par souche ou contient environ 3,8 µg de HA par souche.

6. Composition selon l'une quelconque des revendications précédentes, **caractérisée en ce que** le tocophérol est le DL-α-tocophérol.

7. Composition selon l'une quelconque des revendications précédentes, **caractérisée en ce que** l'émulsion a des gouttelettes ayant un diamètre sub-micronique.

8. Composition selon l'une quelconque des revendications précédentes, **caractérisée en ce que** l'antigène du virus de la grippe est préparé à partir d'un virus de la grippe ayant un ou plusieurs segments d'ARN d'un virus de la grippe A/PR/8/34.

9. Composition selon l'une quelconque des revendications précédentes, **caractérisée en ce que** l'adjuvant comprend un lipide A monophosphorylé 3-O-déacylé (3dMPL).

10. Composition selon la revendication 9, **caractérisée en ce qu'**au moins 10% en poids du 3dMPL est sous forme de chaîne hexaacyle.

11. Composition selon l'une quelconque des revendications précédentes, étant sensiblement dépourvue de matière mercurielle.

12. Composition selon l'une quelconque des revendications précédentes, comportant un ou plusieurs tampons.

13. Composition selon la revendication 12, **caractérisée en ce que** le ou les tampons incluent : un tampon phosphate, un tampon Tris, un tampon borate, un tampon succinate, un tampon histidine, ou un tampon citrate.

14. Composition selon l'une quelconque des revendications précédentes, ayant un pH compris entre 5,0 et 8,1.

15. Composition selon l'une quelconque des revendications précédentes, **caractérisée en ce que** la composition comporte deux souches de grippe A et une souche de grippe B.

16. Composition selon l'une quelconque des revendications 1 à 14, **caractérisée en ce que** la composition est un vaccin monovalent contre une souche pandémique du virus de la grippe.

17. Kit pour la préparation de la composition selon l'une quelconque des revendications précédentes, comprenant (a) un premier composant de kit comprenant un antigène sous-unitaire du virus de la grippe préparé à partir d'un virus cultivé dans une culture cellulaire et ne comportant aucune protéine d'oeuf, et (b) un deuxième composant de kit comprenant un adjuvant Th1 sous forme (i) d'une émulsion huile dans l'eau qui comporte du squalène, un tocophérol et un polysorbate 80, ou (ii) une émulsion sub-micronique de squalène, de polysorbate 80, de trioléate de sorbitane, et d'un oligonucléotide immunostimulateur ; le premier et le deuxième composant étant dans des récipients distincts.
